(19) Europäisches Patentamt / European Patent Office / Office européen des brevets

(11) **EP 2 409 640 A1**

(12) **EUROPEAN PATENT APPLICATION**
published in accordance with Art. 153(4) EPC

(43) Date of publication:
**25.01.2012 Bulletin 2012/04**

(51) Int Cl.:
**A61B 5/024** (2006.01)       **A61B 5/18** (2006.01)
**B60K 28/06** (2006.01)

(21) Application number: **10753583.3**

(22) Date of filing: **18.03.2010**

(86) International application number:
**PCT/JP2010/054703**

(87) International publication number:
**WO 2010/107092 (23.09.2010 Gazette 2010/38)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **18.03.2009 FR 0951715**

(71) Applicant: **Aisin Seiki Kabushiki Kaisha Aichi 448-8650 (JP)**

(72) Inventor: **VRAZIC, Sacha Kariya-shi Aichi 448-8650 (JP)**

(74) Representative: **Kuhnen & Wacker Patent- und Rechtsanwaltsbüro Prinz-Ludwig-Straße 40A 85354 Freising (DE)**

(54) **BIOLOGICAL PARAMETER MONITORING METHOD, COMPUTER PROGRAM, AND BIOLOGICAL PARAMETER MONITORING DEVICE**

(57)      A method for monitoring a biological parameter out of either the heartbeat and/or respiratory signal of an occupant (2) on a member of a seat (8) or bed. The member supports sensors (6), receives the signal from each of the group of sensors, inputs the signal into an atom dictionary, selects some sensors according to the input, and performs monitoring using only the selected sensors.

Also provided is a computer program including code instructions capable of controlling execution of the method of the invention when the method is executed by a computer. Further provided is a monitoring device for monitoring a biological parameter out of either the heartbeat and/or respiratory signal of an occupant on a member of a set or bed.

## FIG. 1

**Description**

Technical Field

The present invention relates in particular, but is not limited, to extraction and monitoring of biological parameters of the occupant of a vehicle, whether the driver or a passenger. In particular, this relates to extracting the heartbeat and/or respiratory signal of a person so as to not be restrictive and if possible under all driving conditions. In fact, by obtaining these biological parameters, a monitoring system can be used for improving road traffic safety. In particular, this is intended to reduce the number of accidents caused by drowsiness or illness symptoms.

Background Art

For example, from Patent Literature 1 a method is known for collecting biological parameters from the occupant of a seat. The seat includes a piezoelectric sensor array capable of collecting signals for extracting the parameter to be monitored. This patent takes into consideration the operator selecting a sensor supplying the most accurate signal relating to the biological parameter.
However, with this operator-based selection mode, automation of the monitoring method is not possible, and moreover, it is impossible to compose an onboard version for constantly monitoring the biological parameters of the driver and/or passengers while driving.

Prior Art Literature

Patent Literature

  Patent Literature 1: U.S. Patent Application Publication No.2008/0103702

Disclosure of Invention

Problem to be Solved by the Invention

It is an object of the present invention to enable automatic monitoring of biological parameters of the occupant of one member, and monitoring by onboard systems. The present invention further aims to improve the quality of monitoring of these parameters.

Means for Solving the Problem

In order to accomplish this, the present invention comprises a method for monitoring biological parameters, the method for monitoring at least one biological parameter out of a heartbeat and/or a respiratory signal of an occupant on a member of a seat or a bed, the method **characterized in that**:

  the member supports sensors;
  a signal is received from each of the sensors in a group;
  the resulting signals are inputted into an atom dictionary (dictionnaire d' atomes in French);
  one or more of the sensors are selected through the inputting; and
  the monitoring is implemented using only the selected one or more sensors.

By inputting the signal into an atom dictionary in this manner, it is possible to select sensors to supply the most appropriate signals for monitoring the biological parameters by an automated means with high reliability. Consequently, this monitoring approach can respond in real time to the characteristics and posture of the occupant, changes in the posture or movement of the occupant. Through this, the method can monitor biological parameters by responding simultaneously both spatially and in time to the various conditions of monitoring without going through an operator. Accordingly, this method promotes the acquisition of the most reliable data relating to the biological parameters that are to be monitored.
Advantageously, the atoms of the dictionary are composed of a combination of g and h, where type g = a.sin and h = b.cos, with a and b being coefficients.
Advantageously, each atom of the dictionary is weighted using a window, and preferably a hanning window.

Advantageously, a value linked to signals in the dictionary, for example the maximum value of the norm of the signals in the dictionary, is determined.

Consequently, this relates to the usage mode of inputting signals into the atom dictionary.

Advantageously, a prescribed number of signals having the strongest value among the signals are identified.

Accordingly, with this embodiment, when p is the prescribed value, the first p signals having the strongest value are selected. Through this, it is guaranteed that a fixed number of signals selected last will be used. As an alternative means, only signals whose values exceed some threshold values may be considered.

Preferably, the following determination is made with regard to each signal. $\sup |C(F, g_\gamma, h_\gamma)|$

Here, f is a signal, $g_\gamma$ and $h_\gamma$ are atom pairs in the dictionary, $\gamma$ is a natural number and C is a distance function. For example, $C(f, g_\gamma, h_\gamma) = \phi_{2,\gamma} \cdot (<f, g_\gamma>^2 + <f, h_\gamma>^2 - 2\phi_{1,\gamma} <f, g_\gamma> \cdot <f, h_\gamma>)$. Here, $\phi_{1,\gamma}$ and $\phi_{2,\gamma}$ are normalization coefficients of the pair $(g_\gamma, h_\gamma)$ of the following type:

[Formula 1]

$$\begin{cases} \phi_{1,\gamma} = <h_\gamma, g_\gamma> \\ \\ \phi_{2,\gamma} = \dfrac{1}{1 - \phi_{1,\gamma}^2} \end{cases}$$

For example, in the case of an actual signal:

[Formula 2]

$$< x, y > = \sum_n x_n y_n$$

Advantageously, when at least one prescribed type of movement by the occupant is detected by an unselected sensor, this one or multiple sensors are added at least temporarily to the group of selected sensors.

In this manner, when movement of the occupant is detected, the map of selected sensors is adapted. Through this, the most appropriate sensor signals can be constantly used regardless of the various movements of the occupant. This temporary addition of sensors is particularly advantageous when the movements of the occupant are slow or the amplitude is short.

Advantageously, when at least one prescribed type of movement by the occupant is detected, an input step and a selection step are repeatedly implemented.

Consequently, in the present embodiment, when the occupant moves an input step and a selection step are repeatedly implemented in order to keep the possibility of using the most appropriate signals at a maximum. The present embodiment is particularly applicable for example in the case of movement with large amplitude or quick movements.

Advantageously, when movement by the occupant is detected, the direction of this movement is determined, and when at least one of the unselected sensors exists in this direction, this one or multiple sensors are at least temporarily selected.

In this manner, sensors with a great opportunity to send in a short time appropriate signals for use in biological parameters are selected in advance. With this composition, it is possible to increase opportunities to use the most appropriate signals.

Furthermore, for example when mounted in a vehicle, the vibration level is comprised so that the bulk of the reliability of this method is dependent on the type of process implemented in order to take surrounding noise into consideration. This problem is not limited to monitoring performed when mounted in a vehicle. For example, when monitoring a patient occupying a bed, there is a possibility that surrounding noise (for example, noise generated by electrical instruments) could impede this monitoring, so as a result, the reliability of the present method is similarly dependent on the quality of the process conducted in order to remove the above-described noise from the signal being used. This problem particularly occurs in medical beds in which the patient is transported and vibrations are created when the bed moves.

Consequently, preferably at least one accelerometer is bound to the member;
a model of a transfer function between at least one signal of an accelerometer or the one accelerometer out of the at least one accelerometer at input, and the signal of the sensor or one sensor out of the multiple sensors at output are determined;
a noise value is estimated using this model; and
the estimated noise value is removed from the sensor signals.

In this manner, this accelerometer or various accelerometers are by definition members for sensing vibrations in particular. Consequently, it is possible to supply a high-fidelity, reference signal of noise relating to vibrations. Furthermore, it is possible to estimate the noise value with high reliability by determining the transfer function model. Consequently, it is possible to remove the bulk of the noise in this signal from the signal to be used. Accordingly, this does not directly remove the signal read by this accelerometer or various accelerometers from the signal to be used, or in other words, subtraction of signals is not accomplished. Using the signal of the accelerometer, the effects of the noise in the signal to be used are modeled and the estimated value of this noise is appropriately extracted from the signal. Through this, it is possible to obtain a useable signal containing no noise in particular. Noise that still remains therein is not an impediment to obtaining signals faithfully showing one or multiple biological parameters to be used.

Advantageously, the one or various signals received from sensors are processed using nonlinear filtering.

Accordingly, use of nonlinear filtering (estimation) is particularly suitable as long as the monitoring phenomenon is applied similar to a nonlinear system and noise characteristics change with time. Through this nonlinear filtering, parameters that cannot be directly observed with the collected signals are estimated, and in addition signals relating to the parameters to be monitored can be easily extracted.

With the present invention, a computer program is obtained that includes a code instruction capable of controlling implementation of the method when executed by a computer or a calculator.

With the present invention, a data recording medium is comprised that includes this kind of program in a recorded state.

The present invention is comprised so that this kind of program can be used and provided over a remote communication network to facilitate downloading.

Finally, the present invention provides a biological parameter monitoring device for monitoring at least one biological parameter out of a heartbeat and/or a respiratory signal of an occupant on a member of a seat or a bed, the device characterizing by including:

sensors supported by the member;
a means for receiving signals from various sensors from at least one sensor group, inputting these signals into an atom dictionary and selecting one or more of sensors through the inputting; and
a means for monitoring one or various parameters using only the selected one or more sensors.

Preferably, determination of whether or not a person is in the seat is made and when there is no person in the seat the process is not executed (this equates for example to cases where an object is placed in the seat).

Other characteristics and advantages of the present invention should become more clear from the following description of the preferred embodiments, which are intended to be illustrative and not limiting, and which are described with reference to the attached drawings.

Brief Description of Drawings

FIG. 1 shows the whole device of an embodiment of the present invention.
FIG. 2 is a side view of a seat to which this device is joined.
FIG. 3 is a drawing showing one of the sensor arrays in the seat of FIG. 2.
FIG. 4 is a graph showing the atoms of the dictionary used in the method of the present invention.
FIG. 5 is a drawing showing modeling of the transfer coefficient in the present embodiment.
FIG. 6 is a drawing showing the transfer coefficient modeled in this manner taken into consideration when implementing the present method.
FIG. 7 is a graph showing the time-elapsed amplitude of the signal prior to extraction of noise and after extraction of the noise caused by the transfer coefficient.
FIG. 8 shows in two curves the heartbeat estimated by the present method and the actual heartbeat, and also shows the permissible range of inputs.

Best Mode for Carrying Out the Invention

Below, the method of the present invention and the preferred embodiment of related devices comprising a complete system for extracting biological information from the body of a person 2 who is the driver or a passenger in a vehicle 4 are described. The present invention aims to monitor biological parameters of a person, such as heartbeat and/or respiratory rhythm. This monitoring is preferably accomplished in various driving conditions and ascertains movement of the body. This relates to obtaining the above-described biological parameters and monitoring such. In particular, regardless of driving conditions, it is desirable for the above-described parameters to be obtained so as to not restrict the affected person. In reality, a monitoring system is obtained that improves road traffic safety by reducing accidents caused by drowsiness or a number of illness conditions through the inputting of information relating to heartbeat and/or respiratory signal.

First, the composition of the system and steps implemented by this method are broadly described. Next, several characteristics of such a composition and the present embodiment are described in detail.

The device includes multiple piezoelectric sensors 6 supported by the seat 8 occupied by the person 2. Regardless of the posture or movement of the occupant, these sensors are configured so that desired signals from the sensors can constantly be obtained. The sensors can detect fluctuations in contact pressure and thus are piezoelectric sensors. The sensors are positioned on the seat part 10 and near the primary surface on the upper part of the back part 12 of the seat 8, and this surface is provided so as to contact the occupant 2. The sensors can also be positioned directly on this surface. In this manner, the sensors receive pressure from the body, and in particular receive fluctuations in pressure originating from the body near the arteries in particular. This case has to do with sensors composed of film or sheets. However, as described below, these sensors detect all types of mechanical vibrations, so the desired signals cannot be directly observed from the output of these sensors.

In this embodiment, the seat 8 contains around 60 sensors but naturally this figure is intended to be illustrative and not limiting. The number of sensors in the seat part 10 can be from 10 to 70, and for example can be 40. The number of sensors in the back part 12 can be from 5 to 50, and for example can be 20. A circuit board used when using this method contains for example 20 sensors, that is to say 15 sensors in the seat part and five sensors in the back part. Accordingly, the circuit board includes around 30% of the sensors in the seat part.

Sensor signal processing uses an analog component and a digital component.

The analog component includes a collection step and a composition step for the amplitudes of the output signals from the sensors 6. The various analog outputs are digitized and then several sensors are automatically selected in accordance with the position of the body 2 in the seat 8. Signals sent from these sensors are later processed and united. The selection step and uniting step of the sensors is implemented multiple times when executing this method so as to appropriately follow and predict movement of the body. Bear in mind that movement of the passengers and driver in the vehicle are typically not of the same type. Movements by passengers are more random and are fewer in number than movements by the driver. With regard to movements by the driver, it is necessary to take into consideration the characteristics of the presence of four drive wheels on the vehicle, hand movements, acceleration, braking, the positioning of the feet for gear changes, and so forth.

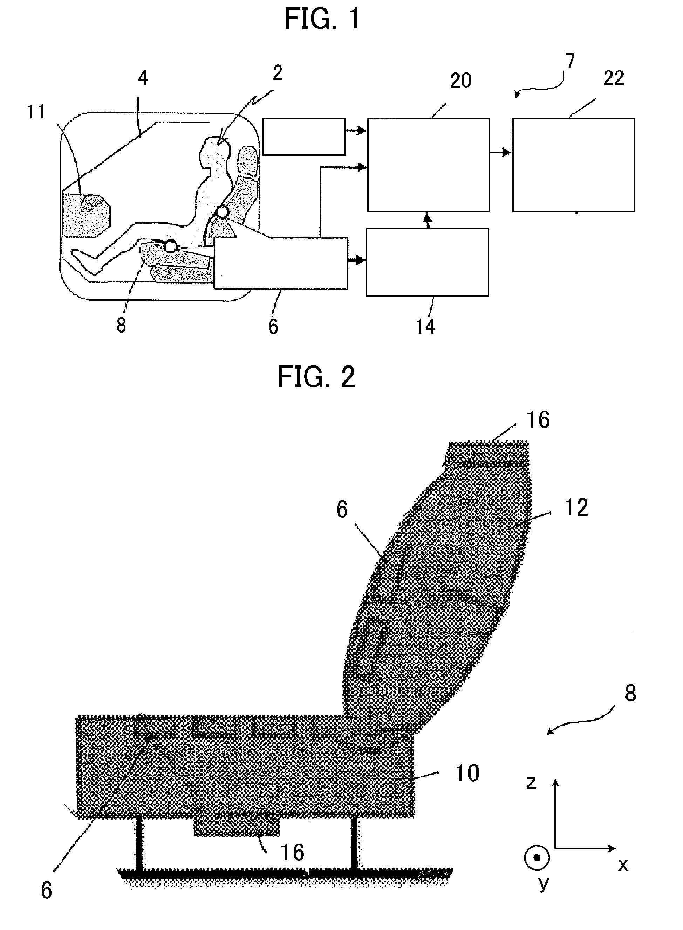

In FIG. 1, a device for accomplishing the roles of selecting and uniting sensor signals is illustrated by a block 14. This block detects movement of the body, and can trace and predict this movement. In particular, this block can select sensors most capable of supplying effective signals for obtaining biological parameters. This block can accurately detect one movement, forecast one movement and obtain a list of candidate sensors that should be selected. Consequently, the present method can be continuously implemented without changes regardless of the occupants' movements. The block can determine whether or not an inert object is placed on the parts, and in such a case does not undertake any process.

The seat 8 is further provided with accelerometers 16 that act particularly as reference sensors for surrounding noise such as vibration noise or the like from the vehicle. These accelerometer sensors 16 each detect noise in three orthogonal directions, that is to say in the horizontal directions X and Y and the vertical direction Z. Within the scope of this method, by estimating transfer coefficients among the accelerometers 16 and the various piezoelectric sensors 6, it is possible to later reduce output noise from these sensors. The transfer coefficients are reestimated whenever reestimation is determined to be necessary for tracing movement of the body and current driving conditions of the vehicle. Consequently, the block 20 fills the role of creating a dynamic model that changes with time in order to estimate transfer coefficients among the accelerometers and the piezoelectric sensors. With the above-described transfer coefficients estimated in this way, it is possible to predict various values relating to noise and to then reduce noise transferred by signals from the piezoelectric sensors. The model used in this step may be either linear or nonlinear. This is the first step in mitigating noise in signals supplied from the sensors 6.

On the other hand, there are times when this noise mitigation step is determined to be insufficient in a number of cases. Consequently, the block 20 contains a second estimation and tracking step for more accurately obtaining biological parameters such as heartbeat and/or respiratory signals. This step can more appropriately track fluctuations in biological parameters regardless of driving conditions (movement of the body, driving in a city, high-speed driving or driving on an expressway). This second step is composed so as to conform to nonlinear systems. This step can include an extended Kalman filter or independent filter so as to better identify all noise fluctuations. Such a nonlinear process step is truly well suited to extracting and monitoring parameters deriving from nonlinear models, such as heartbeat and respiratory rhythm due to a vibration environment that changes with time.

After the two filter steps, a block 22 can obtain the desired signal, monitor this and predict changes therein.

The present invention is suited for all types of vehicles. However, the present invention is not limited to vehicles. Consequently, the present invention can be used with other types of members, such as seats or beds, for example medical beds for monitoring patient parameters.

The method used is self-adaptable.

Next, several characteristics of this method will be described in detail. Here, suppose biological parameters of the driver of a vehicle are being monitored.

1. Selecting and uniting piezoelectric sensors.

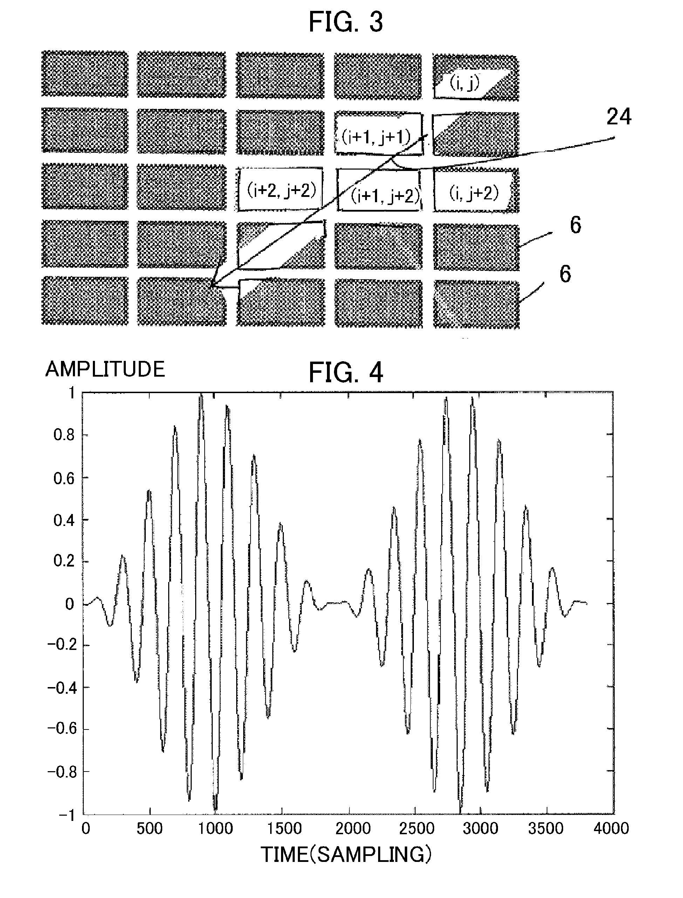

As can be seen from FIGS. 1 through 3, the seat 8 contains two sensor arrays 6 arranged respectively in the seat part 10 and the back part 12. Each of these arrays contains multiple rows and multiple columns in this case. For example, as shown in FIG. 3, the array contains five rows and five columns and forms a checkered pattern of sensors. The sensors in each array are virtually coplanar. The sensors are electrically connected appropriately to other parts of the device, and signals read by these sensors are transferred to means 14 and 20.

The block 14 fills the role of selecting only a number of useable sensors. This for example relates to selecting two sensors in the seat part 10 and two sensors in the back part 12. However, this number can be increased or decreased in accordance with the case.

Suppose the vehicle's engine is off. A driver and passenger enter the vehicle. When the switch is input by an ignition key or comparable part, the device 7 of the present invention including the means 14, 20 and 22 orders use of this method. The process members and calculating member of the device 7 are housed in a dashboard 11, for example.

When use of this method begins, the device 7 causes operation of a default circuit board of sensors 6 basically selected from among the sensors in the seat part and the back part. This circuit board has memory. This default circuit board is part of the standard adjustment of this method.

When the body of the person 2 is in the seat 8, signals are sent from a number of sensors 6. The means 14 estimates whether or not adjustment of the circuit board is desirable by taking into consideration various conditions, in particular the characteristics of the body 2 and the posture thereof in the seat, by analyzing those signals. The means 14 also analyzes whether or not an object exists in the part in place of a person. If such is the case, no tracking process is accomplished.

Consequently, the means 14 basically identifies signals from the sensors 6 supplying signals that are most usable. No consideration is given to sensors supplying absolutely no signals. In addition, no consideration is given to signals from sensors supplying high-pressure signals. This is because with the present method, pressure fluctuations in particular are watched. Accordingly, sensors positioned below the buttocks of the person 2 receive most of the weight of the person's torso and supply signals with relatively poor information. These are in general not taken into consideration. At this step, a circuit board with the greatest correlation to the means 14 must be obtained, in this case a circuit board provided with sensors for detecting slight movements, that is to say slight pressure fluctuations. Consequently, a number of sensors are added or removed, and through this the basic circuit board is adapted. In the circuit board adapted in this manner, selection of sensors is accomplished in later steps of this method.

In later steps of this method, the block 14 is configured to select new sensors that can be correlated to this each time slow body movements or small-amplitude movements are detected. When quick body movements or large amplitudes are detected, the block 14 makes the sensor circuit board completely new and adapts such to conditions most appropriately.

Next, a description will be given for how the block selects the most appropriate sensors in the presence of a prescribed sensor circuit board, that is to say the sensors with the highest probability of including desired signals relating to biological parameters.

a. Selection of signals

Here, simultaneous time-frequency analysis is conducted. Consequently, it is possible to estimate weight using atom decomposition providing high frequency precision and to ascertain the position of components that should be tracked. This is expanded by one type of wavelet packet.

Consequently, one sensor signal is illustrated here as a linear combination of expansion functions $f_{m,n}$.

[Formula 3]

$$x_n = \sum_{m=1}^{M} \alpha_m f_{m,n}$$

This signal x can be expressed as follows using matrix notation.

$x = F\alpha$, where $F = [f_1, f_2, ..., f_M]$.

The signal x here is a column vector (N x 1 formant), $\alpha$ is a column vector of expansion functions (N x 1), and F is an N x M matrix whose columns are expansion functions $f_{m,n}$. A single signal model is supplied by a single linear combination of expansion coefficients and various functions. Compact multiple models tend to include expansion functions that have a large correlation to the signal.

Preparing an atom dictionary suitable to wide-ranging time-frequency behavior, it is possible to break down signals by selecting a number of suitable atoms from the atom dictionary. This dictionary is comprised as follows.

The pulse response of piezoelectric sensors is known to be a decreasing sine waveform accompanying basic frequency offset. Hence, in order to make it possible to cover all phases, if the dictionary is comprised of vectors of the type $D = (g_\gamma, h_\gamma)$ when $g_\gamma$ is a cosine waveform and $h_\gamma$ is a sine waveform, an extremely adaptable dictionary can be obtained.

In this manner, the dictionary is comprised of sine waveforms and cosine waveforms of various frequencies (here, the frequencies are limited to within the monitoring range of the present invention). In this case, because the strongest frequency is 20Hz, in this embodiment it is preferable to use a frequency range of 0.2Hz to 3Hz for a single respiratory signal. For two signals combining a respiratory signal and a heartbeat, a range from 0.7 to 20 Hz is used, and in all cases, one pitch is 0.1 Hz.

In order to obtain a sufficient frequency resolution, each atom in the dictionary is weighted by a hanning window, and through this it is possible to avoid an edge effect in particular. Consequently,

[Formula 4]

$$\begin{cases} h_\gamma = w.\sin 2\pi\gamma k \\ g_\gamma = w.\cos 2\pi\gamma k \end{cases}$$

Here,

[Formula 5]

$$w = \frac{1}{2}(1 - \cos(2\pi(1:m)/m+1))$$

Here, m indicates the length of the atom. In fact, this length is an important value in frequency resolution. When this weighting is not present, atoms could possibly have continuity times differing in the dictionary.

Accordingly, the dictionary is composed of N weighted sine atoms and N weighted cosine atoms. Consequently, these atoms form compact (that is to say limited, that is to say composed of a non-zero, limited number of points) support signals that can be viewed the same as wavelet packets by analogy.

Accordingly, FIG. 4 shows one atom of the dictionary surely containing the combination of one sine atom and one cosine atom for one frequency. The signal is shown with time on the horizontal axis and amplitude on the vertical axis. The length of the atom can be measured between the point of 0 samples and the point of 2000 samples on the horizontal axis, and time is selected as the sample number (dependent on the sampling frequency).

Furthermore, a normalization coefficient is calculated for each group of atoms.

[Formula 6]

$$\begin{cases} \phi_{1,\gamma} = <h_\gamma, g_\gamma> \\ \\ \phi_{2,\gamma} = \dfrac{1}{1 - \phi_{1,\gamma}^2} \end{cases}$$

Through this, it is possible to compare atoms having originally differing weights and lengths.

In this manner, a dictionary forming a normal orthogonalized base can be obtained.

The signal f supplied from each sensor of the circuit board (in other words, that pulse response), is input into each group of the atom dictionary and a value is calculated as follows:

$$\sup |C(f, g_\gamma, h_\gamma)|$$

Here, $C(f, g_\gamma, h_\gamma)$ is a distance function. In this example, the following distance function is selected:

$$C(f, g_\gamma, h_\gamma) = \phi_{2,\gamma} \cdot (<f, g_\gamma>^2 + <f, h_\gamma>^2 - 2\phi_{1,\gamma} <f, g_\gamma> \cdot <f, h_\gamma>).$$

With this distance function, the position of the component generated simultaneously by the person's body and the system (resonance between the weight of the body and vibrations of the vehicle) can be accurately ascertained. Consequently, when a signal transferred by sensors simultaneously includes system parameters and the person's biological parameters, the sensor is considered to be competent. When only system parameters are included, this is considered to be incompetent.

In this case, the values calculated for the signals are classified in order of size, and a list of competent sensors is created. A natural number p is determined in advance, and the first p values of this list are considered. The first p sensors corresponding to these values are the selected sensors.

b. Predicting movement

Because this method appropriately retains sets of related signals (biological parameters of the occupant), it is possible to predict movements of the person's body. Consequently, in this example the following approach is used.

The generation of a body's movement is detected by sensors of the circuit board where supplied signals begin fluctuating. In FIG. 3, sensors of the circuit board on the back part are assumed to be sensors identified by these references (i, j), (i+1, j+1), and (i, j+2).

The means 14 identifies movement through these sensors and this same means can predict the direction of this movement indicated by the arrow 24 in FIG. 3 through interpolation. Consequently, while this movement is being conducted, the means 14 predicts future movement, and appends sensors capable of supplying beneficial signals during future movements in the path of the movement to the circuit board of selected sensors during the movement. In FIG. 3, these are the two sensors (i+2, j+2) and (i+1, j+2), which are in the same row as the sensor (i, j+2). Accordingly, the means 14 can take into consideration as quickly as possible anticipated signals supplied from these sensors. Following this, when it is confirmed that movement at the positions of these sensors is recognized, these sensors are retained in the circuit board. In contrast, when there is an error in prediction and no movement is detected by at least one of the sensors, that sensor is removed from the circuit board.

2. Transfer function identification and estimation

In order to fill the role of a standard for vibration noise in the vehicle, multiple accelerometers 16 are used. As long as the vehicle vibrations are not influenced in a single direction, for example as long as these are not influenced only in the vertical direction, ideally three axes or 3D accelerometers are used. Furthermore, it is preferable to use at least two accelerometers.

There are cases where it is judged that specifying the positions of these accelerometers is important in order to obtain a highly reliable model. For example, by positioning one accelerometer 16 on the bottom of the seat part in the structure of the seat part 10 of the seat, this accelerometer detects vibrations of the occupant at the bottom of the seat. In this embodiment, a second accelerometer is positioned at the top of the back part 12. This is because this part of the seat has a certain independence from the seat part, so vibrations can be confirmed.

In accordance with the positioning of the piezoelectric sensors 6 and the accelerometers 16, it is possible to make modeling of the transfer function implemented following that linear or nonlinear. In either case, parameters of this modeling are estimated by recursive ordering in this case. Furthermore, the above-described parameters are from time to time reestimated during implementation of this method so that the model is accurately applied to various conditions, in particular driving conditions.

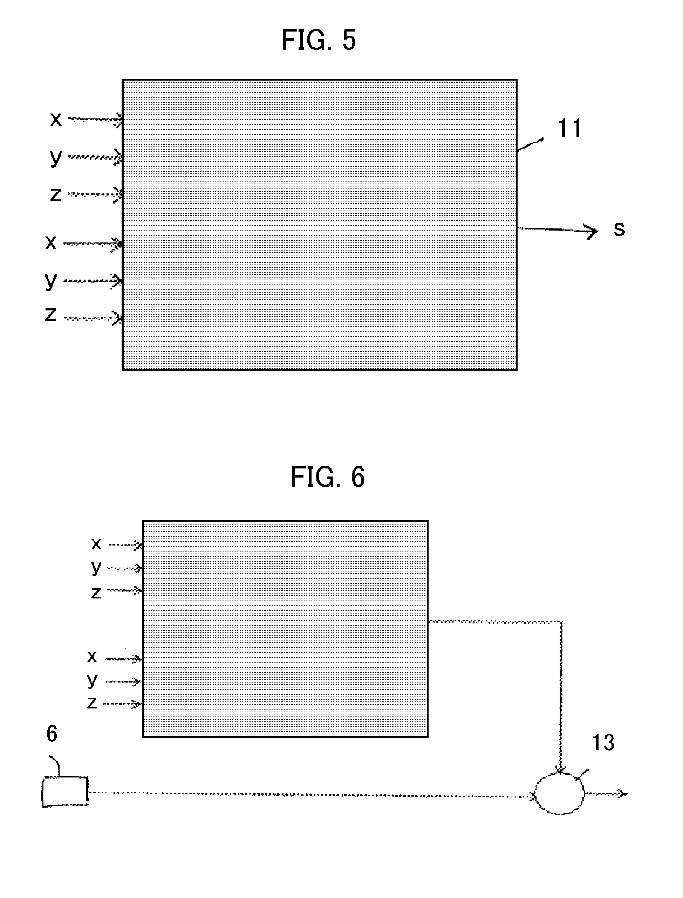

The transfer function is modeled for each of the selected piezoelectric sensors 6. Consequently, as shown in FIG. 5, this transfer function has an output signal for all accelerometers 16 at the input. In this case, this is related to the three signals corresponding to vibrations in the X, Y and Z directions, respectively, supplied by the accelerometers 16 in the seat part, and three similar signals supplied by the accelerometers 16 in the back part. The transfer function has a signal s supplied by the piezoelectric sensors 6 taken into consideration, at the output. Consequently, the principle of modeling the transfer function is shown in FIG. 5. This concerns the function 11 and identification of those parameters, and has at the input the x, y and z signals supplied by the two accelerometers and at the output supplies the signal s supplied by the piezoelectric sensors taken into consideration. In this manner, modeling of the unique effects of vibrations in the signals supplied by the piezoelectric sensors is obtained.

In this case, the means 20 first determines the optimum type of model in accordance with the conditions in order to obtain a transfer function from among a list of multiple types of models. This list is as follows in this case:

    modeling by indicating condition,
    ARMA,
    ARX,
    NLARX.

After testing modeling with each type of model, basically the type of model that is most suitable is taken into consideration.

Next, as shown in FIG. 6, the noise value is dynamically determined in accordance with intermittent signals supplied by the accelerometers, using the model identified in this manner. Input is six signal values from the accelerometers. Output is an estimated value by simple noise on the signal side of the piezoelectric sensors. In this case, this estimated value is subtracted from the signal supplied from the piezoelectric sensors 6 at the position of a subtracter 13. After this subtraction a signal is obtained in which a large portion of the effects of vibration noise have been removed.

In the present embodiment, the means 20 uses as a default an ARX-type external self-recursive model. This means that when better results cannot be obtained from any of the other types of models in the list, this type of model is used. When this is not the case, a model that supplies the best results is used. The structure of this model is as follows:

[Formula 7]

$$A(q) \cdot y(t) = \sum_{1}^{Ni} B_i(q) \cdot u_i(t - n_{ki}) + e(t)$$

Here, A(q) is a polynomial having $N_A$ coefficients, y(t) is the output signal of a piezoelectric sensor, $B_i$(q) is a polynomial having $N_B$ coefficients, $u_i$(t) (i=1, ..., $N_i$) is an input signal supplies from an accelerometer, $n_{ki}$ is a unit delay number in the input and e(t) is an error signal of this model.

The total number of free coefficients $N_C$ is as follows:

$$N_C = N_A + N_i \cdot N_B$$

The polynomial coefficients are estimated by minimizing the trace of an error prediction covariance matrix. As explained above, such estimation of the parameters is updated at times with changes in driving conditions. When parameters of the model are estimated by each sampling step, predicted noise in the piezoelectric sensors (in other swords, estimated noise) can be calculated. In this case, this estimated noise is removed by the output of the piezoelectric sensors as shown in FIG. 6.

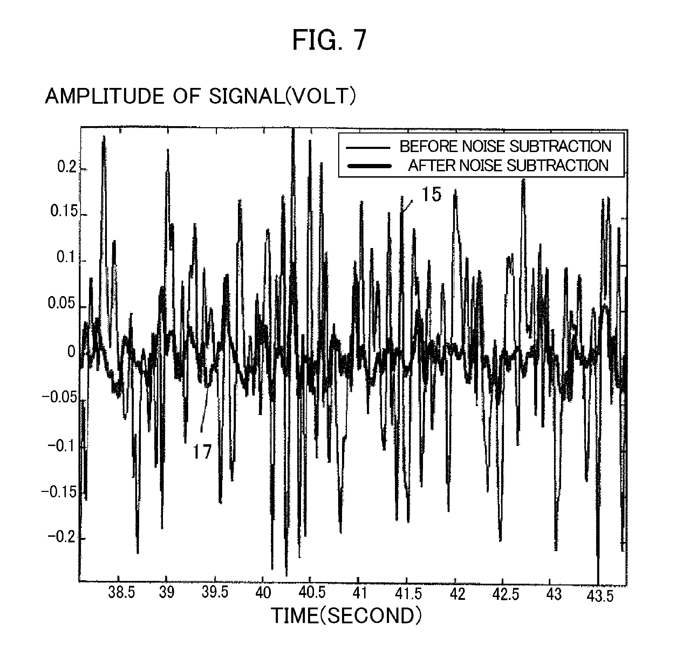

In FIG. 7, the signal 15 of the piezoelectric sensor 6 prior to subtraction is shown by the thin line and the signal 17 after subtraction is shown by the bold line. In particular, the amplitude (units: volts) of the signal on the vertical coordinate is dramatically reduced after subtraction, so that peaks corresponding to heartbeat appear clear.

3. Extraction of biological parameters

After this noise removal step, it is necessary to extract the heartbeat and respiratory signals using the means 20. This returns to estimating parameters for frequencies that cannot be directly observed. Accordingly, positioning in Bayes estimation is particularly effective. Furthermore, because the system being discussed is a nonlinear type, it is possible to use an extended Kalman filter. An independent filter may also be used in order to more closely recognize noise fluctuations that are not Gauss noise.

As an example, use of an extended Kalman filter is shown in order to estimate and monitor heartbeats.

Here, modeling of piezoelectric sensor signals responding to blood pressure as the sum of sinusoidal high-frequency components having a slowly changing amplitude element and a phase element is proposed.

[Formula 8]

$$y(t) = \sum_{i=1}^{m} a_i(t) \cdot \sin \phi_i(t)$$

Here, $\phi_1(t) = \omega(t) \cdot t$,

$$\phi_i(t) = i \cdot \omega(t) \cdot t + \theta_i(t), \text{ where } i = 2, \ldots, m;$$

$\omega(t)$ indicates the basic pulse of the signal related to the heartbeat,

m is the number of sinusoidal waveform components,

$a_i(t)$ indicates the amplitude of the sinusoidal waveform component,

$\phi_i(t)$, where i = 2, .., m, indicates the intermittent phase of the higher modulation wave, and

$\theta_i(t)$ indicates the phase difference between the basic pulse and the higher modulation wave.

From this equation, a vector having the below status is proposed.

[Formula 9]

$$\hat{\mathbf{x}}_k = \begin{bmatrix} \omega_k \\ a_{k,1} \\ \vdots \\ a_{k,m} \\ \phi_{k,1} \\ \vdots \\ \phi_{k,m} \end{bmatrix}$$

The change with time in the amplitude $a_{k,i}$ of the sine component is modeled as follows through additional white Gauss noise.

$$a_{k+1,i} = a_{k,i} + v_{k,i}^{a}$$

The change with time in the intermittent basic pulse $\omega_k$ can also be modeled through additional white Gauss noise.

$$\omega_{k+1} = \omega_k + v_k{}^\omega$$

The same is true with regard to the phase difference $\theta_i(t)$ between the basic pulse and the higher modulation wave component, and the change with time in the intermittent phase $\phi_{k,i}(t)$ can be obtained from the following equation.

$$\phi_{k+1,i} = i \cdot \omega_k + \phi_{k,i} + v_{k,i}{}^\phi$$

This kind of selection means that $\omega_k$ is expressed as the ratio between the actual pulse and the sampling frequency of the signal. As a result, the formula indicating the state transition is linear and can be given by the following equation.

[Formula 10]

$$\mathbf{x}_{k+1} = \mathbf{F}(\mathbf{x}_k, \mathbf{v}_k) = \begin{bmatrix} \omega_k \\ a_{k,1} \\ \vdots \\ a_{k,m} \\ 1 \cdot \omega_k + \phi_{k,1} \\ \vdots \\ m \cdot \omega_k + \phi_{k,m} \end{bmatrix} + \mathbf{v}_k$$

This can also be written as follows:

$$x_{k+1} = A \cdot x_k + v_k \qquad (1)$$

Here,

[Formula 11]

$$\mathbf{A} = \begin{bmatrix} 1 & & & & & \\ & 1 & & & & \\ & & \ddots & & & \\ & & & 1 & & \\ 1 & & & & 1 & \\ \vdots & & & & & \ddots \\ m & & & & & 1 \end{bmatrix} \quad (2)$$

Estimation of the dispersion of components of the noise $v_k$ has an effect on the speed of change in the estimated parameters (pulse, amplitude component and phase component) and the convergence speed of the algorithm.

By taking equation (1) into consideration, the formula showing basically predicted observations can be given by the following equation.

[Formula 12]

$$\hat{\mathbf{y}}_k^- = \mathbf{H}(\hat{\mathbf{x}}_k^-, \mathbf{w}_k) = \sum_{i=1}^{m} \hat{a}_{i,k} \cdot \sin \hat{\phi}_{i,k} + \mathbf{n}_k \quad (3)$$

The equation indicating observation is nonlinear, and through this use of an extended Kalman filter is supported. The distribution $n_k$ of observed noise is related to the distribution of noise observed with the piezoelectric signal.

The algorithm of the extended Kalman filter can be executed as follows.

Initialization step:

[Formula 13]

$$\hat{\mathbf{x}}_0 = \mathrm{E}[\mathbf{x}_0]$$

$$\mathbf{P}_{\mathbf{x}_0} = \mathrm{E}\left[\left(\mathbf{x}_0 - \hat{\mathbf{x}}_0\right)\cdot\left(\mathbf{x}_0 - \hat{\mathbf{x}}_0\right)^T\right]$$

When $k \in \{1, ..., \infty\}$, the prediction equation of the extended Kalman filter is as follows:

[Formula 14]

$$\hat{\mathbf{x}}_k^- = \mathbf{F}(\hat{\mathbf{x}}_{k-1}, \overline{\mathbf{v}})$$

$$\mathbf{P}_{\mathbf{x}_k}^- = \mathbf{A}_{k-1}\cdot\mathbf{P}_{\mathbf{x}_{k-1}}\cdot\mathbf{A}_{k-1}^T + \mathbf{W}_k\cdot\mathbf{Q}^{\mathbf{w}}\cdot\mathbf{W}_k^T$$

The updated equation is as follows:

[Formula 15]

$$\mathbf{K}_k = \mathbf{P}_{\mathbf{x}_k}^-\cdot\mathbf{C}_k^T\cdot\left(\mathbf{C}_k\cdot\mathbf{P}_{\mathbf{x}_{k-1}}^-\cdot\mathbf{C}_k^T + \mathbf{V}_k\cdot\mathbf{R}^v\cdot\mathbf{V}_k^T\right)^{-1}$$

$$\hat{\mathbf{x}}_k = \hat{\mathbf{x}}_k^- + \mathbf{K}_k\cdot\left(\mathbf{y}_k - \mathbf{H}(\hat{\mathbf{x}}_k^-, \mathbf{w}_k)\right)$$

$$\mathbf{P}_{\mathbf{x}_k} = \mathbf{C}_k^T\cdot\left(\mathbf{I} - \mathbf{K}_k\cdot\mathbf{C}_k\right)\cdot\mathbf{P}_{\mathbf{x}_k}^-$$

Here,

[Formula 16]

$$A_k \overset{\Delta}{=} \left. \frac{\partial F(x, \overline{v})}{\partial x} \right|_{\hat{x}_k} \, , \quad W_k \overset{\Delta}{=} \left. \frac{\partial F(\hat{x}_k^-, v)}{\partial v} \right|_{\overline{v}} \, ,$$

$$C_k \overset{\Delta}{=} \left. \frac{\partial H(x, \overline{n})}{\partial x} \right|_{\hat{x}_k} \, , \quad V_k \overset{\Delta}{=} \left. \frac{\partial H(\hat{x}_k^-, n)}{\partial n} \right|_{\overline{n}}$$

In addition, here $Q^w$ and $R^\eta$ are the common dispersion matrices of $v_k$ and $n_k$, respectively, and I is the unit matrix.

Accordingly, this is a standard algorithm related to the extended Kalman filter.

In the case of the present invention, this algorithm is applied as follows. The value of noises

[Formula 17]

$$\overline{v} = E[v]$$

and

[Formula 18]

$$\overline{n} = E[n]$$

are assumed to be equal to zero.

Equation (1), which shows status migration, is linear, and because this is known, the following results.

[Formula 19]

$$\mathbf{A}_k \overset{\Delta}{=} \left. \frac{\partial \mathbf{F}(\mathbf{x}, \overline{\mathbf{v}})}{\partial \mathbf{x}} \right|_{\hat{\mathbf{x}}_k} = \mathbf{A} \quad (4)$$

Here, A is obtained from Equation (2).

Taking equations (1) and (3) into consideration, the following results.

[Formula 20]

$$\mathbf{W}_k \overset{\Delta}{=} \left. \frac{\partial \mathbf{F}(\hat{\mathbf{x}}_k^-, \mathbf{v})}{\partial \mathbf{v}} \right|_{\overline{\mathbf{v}}} = \mathbf{I}^{2 \cdot m + 1}$$

and

[Formula 21]

$$\mathbf{V}_k \overset{\Delta}{=} \left. \frac{\partial \mathbf{H}(\hat{\mathbf{x}}_k^-, \mathbf{n})}{\partial \mathbf{n}} \right|_{\overline{\mathbf{n}}} = 1 \quad (5)$$

Finally, equation (3) becomes as follows.

[Formula 22]

$$\mathbf{C}_k \overset{\Delta}{=} \frac{\partial \mathbf{H}(\mathbf{x}, \overline{\mathbf{n}})}{\partial \mathbf{x}}\bigg|_{\hat{\mathbf{x}}_k} = \begin{bmatrix} 0 & \sin \hat{\phi}_{k,1}^- & \dots & \sin \hat{\phi}_{k,m}^- \\ \hat{a}_{k,1}^- \cdot \cos \hat{\phi}_{k,1}^- & \dots & \hat{a}_{k,m}^- \cdot \cos \hat{\phi}_{k,m}^- \end{bmatrix} \quad (6)$$

In order to simultaneously manipulate multiple signals from piezoelectric sensors, it is possible to also enlarge the size of the condition vector and the observation vector. For example, by using two signals from sensors, the condition vector, condition migration matrix and linear matrix showing observation become as follows.

[Formula 23]

$$\hat{\mathbf{x}}_k = \begin{bmatrix} \omega_k \\ a_{k,1,1} \\ \vdots \\ a_{k,1,m} \\ a_{k,2,1} \\ \vdots \\ a_{k,2,m} \\ \phi_{k,1,1} \\ \vdots \\ \phi_{k,1,m} \\ \phi_{k,2,1} \\ \vdots \\ \phi_{k,2,m} \end{bmatrix}, \ \mathbf{A} = \begin{bmatrix} 1 & & & & & & & & & & & \\ & 1 & & & & & & & & & & \\ & & \ddots & & & & & & & & & \\ & & & 1 & & & & & & & & \\ & & & & 1 & & & & & & & \\ & & & & & \ddots & & & & & & \\ & & & & & & 1 & & & & & \\ 1 & & & & & & & 1 & & & & \\ \vdots & & & & & & & & \ddots & & & \\ m & & & & & & & & & 1 & & \\ 1 & & & & & & & & & & 1 & \\ \vdots & & & & & & & & & & & \ddots \\ m & & & & & & & & & & & & 1 \end{bmatrix},$$

$$\mathbf{C}_k = \begin{bmatrix} 0 & & 0 \\ \sin \hat{\phi}_{k,1,1}^- \\ \\ \sin \hat{\phi}_{k,1,m}^- \\ \hat{a}_{k,1,1}^- \cdot \cos \hat{\phi}_{k,1,1}^- \\ \\ \hat{a}_{k,1,m}^- \cdot \cos \hat{\phi}_{k,1,m}^- \\ & \sin \hat{\phi}_{k,2,1}^- \\ \\ & \sin \hat{\phi}_{k,2,m,}^- \\ & \hat{a}_{k,2,1}^- \cdot \cos \hat{\phi}_{k,2,1}^- \\ \\ & \hat{a}_{k,2,m}^- \cdot \cos \hat{\phi}_{k,2,m}^- \end{bmatrix}^T \quad (7)$$

In this process, it is possible to process one or multiple piezoelectric sensors in order to find the heartbeat (and/or respiratory signal). This model, which shows conditions, is cited as one example and is not intended to be limiting.

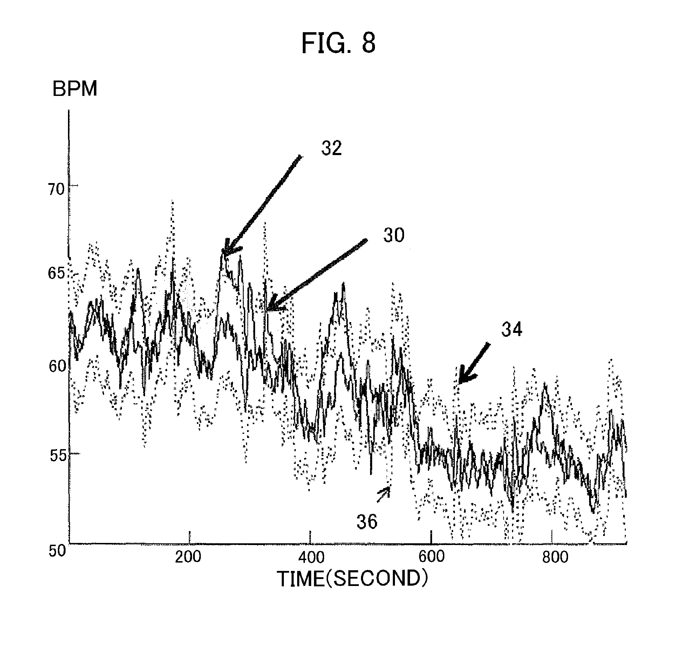

The results of the above process are shown in FIG. 8 as an example. Actual heartbeat signals are shown with a curve 30, heartbeat signals estimated by the method of the present invention are shown with a curve 32 and the tolerance threshold value of $\pm5\%$ in the amplitude of the actual signal is shown by curves 34 and 36. These curves show with the vertical coordinate the pulse number per each minute in accordance with the time (units: seconds) indicated by the horizontal coordinate. Discrepancies between the actual signal and the signal estimated by the method of the present invention are understood to be frequently contained in the tolerance range of $\pm5\%$ of the actual signal. This relates to the test results implemented by actual running conditions (city driving, expressway driving, and so forth).

The means 14, 20 and 22 include a calculation means such as a microprocessor and for example include one or multiple computers having one or multiple memories. The method of the present invention can be automatically implemented through a computer program recorded on a data recording medium such as a hard disk, flash memory, CD or DVD disc. The computer program includes code instructions that can control implementation of the method of the present invention during execution by the computer. Downloading such a program can be comprised so as to provide use via remote communication networks in order to download the latest program version.

Naturally, the present invention can be subjected to numerous modifications without departing from the scope thereof.

Above, an example was described in which a selection step 1, a noise removal step 2 using transfer functions and a nonlinear filtering step 3 are executed in series. As evidenced by FIG. 8, extremely good results are obtained through this series. However, particularly in environments with not very much noise, an arbitrary single step out of these can be used while obtaining acceptable results, and furthermore, it is also possible to use an arbitrary two out of these steps.

In the first step, it is possible to link a unique atom dictionary to each frequency range, and accordingly, in this case it is possible to use two atom dictionaries.

The present invention is based on French Patent Application No.0951715, filed March 18, 2009, and the specification, claims and drawings of French Patent Application No.0951715 are incorporated by reference herein.

**Claims**

1. A method for monitoring biological parameters, the method for monitoring at least one biological parameter out of a heartbeat and/or a respiratory signal of an occupant on a member of a seat or a bed, the method **characterizing in that**:

   the member supports sensors;
   a signal is received from each of the sensors in a group;
   the resulting signals are inputted into an atom dictionary;
   one or more of the sensors are selected through the inputting; and
   the monitoring is implemented using only the selected one or more sensors.

2. The method according to Claim 1, wherein a value linked to signals in the dictionary, for example the maximum value of the norm of the signals in the dictionary, is determined.

3. The method according to Claim 1 or Claim 2, wherein a prescribed number of signals having the strongest value among the signals are identified.

4. The method according to any one of Claims 1 through 3, wherein when at least one prescribed type of movement by the occupant is detected by an unselected sensor, this one or multiple sensors are added at least temporarily to the group of selected sensors.

5. The method according to any one of Claims 1 through 4, wherein when at least one prescribed type of movement by the occupant is detected, an input step and a selection step are repeatedly implemented.

6. The method according to any one of Claims 1 through 5, wherein when movement by the occupant is detected, the direction of this movement is determined, and when at least one of the unselected sensors exists in this direction, this one or multiple sensors are at least temporarily selected.

7. The method according to any one of Claims 1 through 6, wherein at least one accelerometer is bound to the member; a model of a transfer function between at least one signal of an accelerometer or the one accelerometer out of the at least one accelerometer at input, and the signal of the sensor or one sensor out of the multiple sensors at output is determined;

a noise value is estimated using this model; and

the estimated noise value is removed from the sensor signals.

8. The method according to any one of Claims 1 through 7, wherein the one or various signals received from sensors are processed using nonlinear filtering.

9. The method according to any one of Claims 1 through 8, wherein the seat is a seat in a vehicle.

10. A computer program **characterized in that** when executed by a computer or a calculator, a code instruction is included capable of controlling implementation of the method according to any one of Claims 1 through 9.

11. A biological parameter monitoring device for monitoring at least one biological parameter out of a heartbeat and/or a respiratory signal of an occupant on a member of a seat or a bed, the device **characterizing by** including:

sensors supported by the member;

a means for receiving signals from various sensors from at least one sensor group, inputting these signals into an atom dictionary and selecting one or more of sensors through the inputting; and

a means for monitoring one or various parameters using only the selected one or more sensors.

# FIG. 1

# FIG. 2

## FIG. 3

## FIG. 4

AMPLITUDE

TIME(SAMPLING)

# FIG. 5

# FIG. 6

# FIG. 7

AMPLITUDE OF SIGNAL(VOLT)

TIME(SECOND)

FIG. 8

<table>
<tr><td colspan="2">**INTERNATIONAL SEARCH REPORT**</td><td colspan="2">International application No.<br>PCT/JP2010/054703</td></tr>
</table>

A.  CLASSIFICATION OF SUBJECT MATTER
*A61B5/00*(2006.01)i, *A61B5/11*(2006.01)i

According to International Patent Classification (IPC) or to both national classification and IPC

B.  FIELDS SEARCHED

Minimum documentation searched (classification system followed by classification symbols)
A61B5/00, A61B5/11

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| A | JP 2008-113797 A  (Aisin Seiki Co., Ltd.),<br>22 May 2008 (22.05.2008),<br>paragraph [0025]; fig. 1, 2<br>(Family: none) | 1-11 |
| A | JP 2007-501555 A  (Koninklijke Philips Electronics N.V.),<br>25 January 2007 (25.01.2007),<br>paragraph [0005]<br>& US 2006/0209963 A1   & EP 1654706 A<br>& WO 2005/013201 A1   & KR 10-2006-0060000 A<br>& CN 1833259 A | 1-11 |

☒  Further documents are listed in the continuation of Box C.     ☐  See patent family annex.

| | | |
|---|---|---|
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search<br>24 May, 2010 (24.05.10) | Date of mailing of the international search report<br>01 June, 2010 (01.06.10) |
|---|---|
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2010/054703

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2008-79931 A  (Aisin Seiki Co., Ltd.),<br>10 April 2008 (10.04.2008),<br>paragraph [0002]<br>& US 2010/0056937 A1     & WO 2008/038501 A1<br>& DE 112007002169 T | 1-11 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080103702 A
- FR 0951715